# EUROPEAN PATENT APPLICATION

(11) **EP 4 434 645 A1**
(43) Date of publication of application: **25.09.2024**
(21) Application number: 24164062.2
(22) Date of filing: 18.03.2024
(51) Int. Cl.: B08B 9/28, A61L 2/14

(54) **DISPENSING MACHINE AND DISPENSING METHOD TO DISPENSE IONISED AIR INTO A CONTAINER**

(30) Priority: 20.03.2023 IT 202300005166
(71) Applicant: Jonix S.p.A., 35020 Tribano (PD) (IT)
(72) Inventor: CURSI, Lamberto, 35020 TRIBANO (PD) (IT); GAMBINERI, Francesca, 35020 TRIBANO (PD) (IT); CECCHI, Antonio, 35020 TRIBANO (PD) (IT)
(74) Representative: Studio Torta S.p.A.

(57) **Abstract**

A dispensing machine (1) to dispense ionised air into a container (2) having, at the top, a mouth (4). The dispensing machine (1) has: an ionised air generator (13) configured to generate an ionised air flow; a delivery pipe (9) which ends with a delivery opening (10) and is connected to the ionised air generator (13) so as to receive the ionised air flow and emit the ionised air flow from the delivery opening (10); a return pipe (11) which starts with a return opening (12); and an interface element (7) which is configured to be coupled to the mouth (4) of the container (2) and is crossed by the delivery pipe (9) and the return pipe (11) so that, when the interface element (7) is coupled to the mouth (4) of the container (2), the delivery opening (10) of the delivery pipe (9) and the return opening (12) of the return pipe (11) are both inside the container (2).

## Description

### CROSS-REFERENCE TO RELATED APPLICATIONS

This patent application claims priority from Italian patent application no. 102023000005166 filed on March 20, 2023, the entire disclosure of which is incorporated herein by reference.

### FIELD OF THE ART

The present invention relates to a dispensing machine and a dispensing method to dispense ionised air into a container.

The present invention finds advantageous application in the dispensing of ionised air inside a water bottle, to which the following discussion will make explicit reference without the loss of generality.

### PRIOR ART

More and more people are deciding to renounce the use of polluting plastic bottles to use reusable water bottles.

However, it is necessary to regularly clean the inside of a reusable bottle, and the internal cleaning cannot involve just rinsing with tap water: steel, aluminium or glass bottles can become contaminated with algae and microorganisms which find an optimal environment in the presence of water or moisture.

Sanitising the inside of a water bottle can be a relatively time-consuming and laborious operation, especially when the water bottle is used in a working environment where a kitchen and the relative tools are not available.

Utility model CN206689163U and patent US10081864B2 describe dispensing machines provided with an ionised air generator which is connected to a pipe configured to emit an ionised air flow into the bottle.

### DISCLOSURE OF THE INVENTION

The purpose of the present invention is to provide a dispensing machine and a dispensing method to dispense ionised air into a container to enable a fast and effective internal sanitisation of the container.

According to the present invention, a dispensing machine and a dispensing method to dispense ionised air into a container are provided, as claimed by the appended claims.

The claims describe preferred embodiments of the present invention forming an integral part of the present description.

### BRIEF DESCRIPTION OF THE DRAWINGS

The present invention will now be described with reference to the accompanying drawings, which show a non-limiting embodiment thereof, wherein
- figure 1 is a schematic view of a dispensing machine to dispense ionised air into a container and made in accordance with the present invention;
- figure 2 is a schematic view of the dispensing machine of figure 1 in use; and
- figures 3 and 4 are two schematic views of two respective variants of the dispensing machine of figure 1.

### PREFERRED EMBODIMENTS OF THE INVENTION

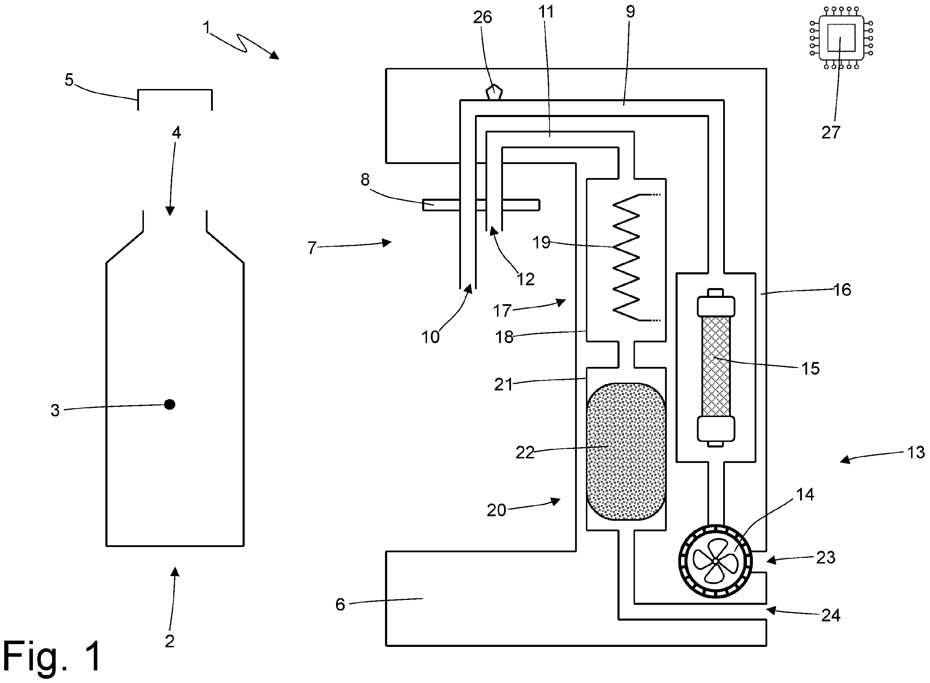

In figure 1, the number 1 denotes a dispensing machine to dispense ionised air into a container 2 having an internal volume 3 intended to contain a liquid (in particular a beverage).

In particular, the container 2 is (in the non-limiting embodiment illustrated in the accompanying drawings) a reusable bottle intended to contain water or other beverages for human consumption; of course, the container 2 could alternatively be any other type of container such as a blister container, a water bag, a thermos, a glass, a bottle, a container for medical, pharmaceutical or food use.

The container 2 has a mouth 4 at the top, which forms the communication towards the exterior of the internal volume 3 of the container 2; thus a liquid (water or other beverage) can be fed into the container 2 through the mouth 4 to fill the container 2 and a liquid present inside the container 2 can be forced out of the container 2 through the mouth 4. Normally, the mouth 4 is closed by a cap 5 which can be screwed onto the container 2 or force-fitted in the container 2.

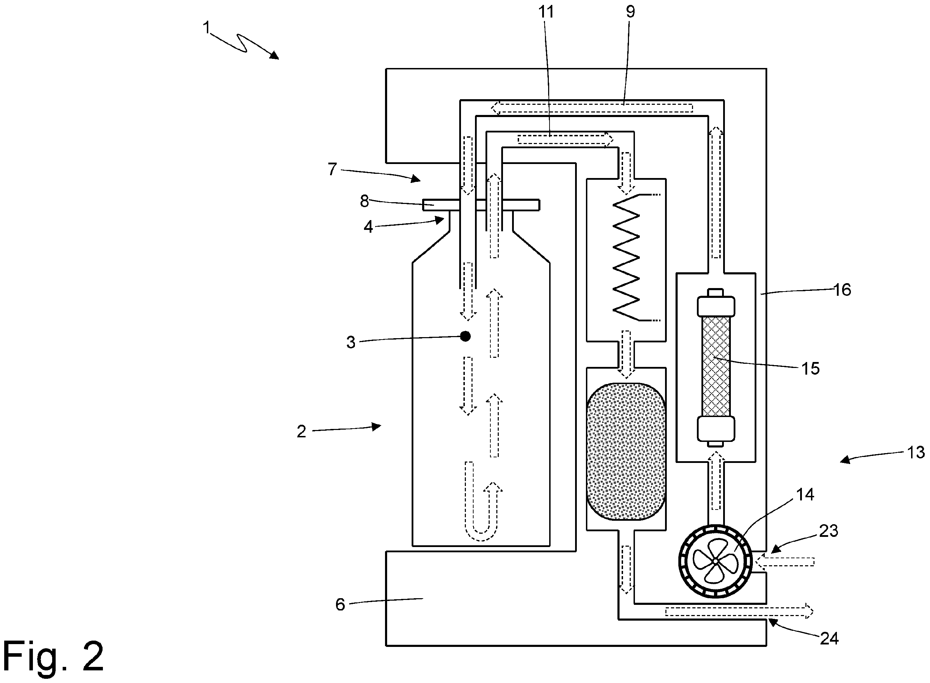

The dispensing machine 1 comprises a frame 6 which is shaped like a 'C' to delimit a space therein which is accessible from the exterior and allows the container 2 to be housed (as illustrated in figure 2). An upper portion of the frame 6 houses an interface element 7 which is configured to couple (rest against) the mouth 4 of the container so as to engage the mouth 4 without appreciable clearance, i.e., to temporarily plug (close so as to eliminate any slit) the mouth 4.

According to a preferred embodiment, the interface element 7 comprises a flat wall 8, which is elastically deformable and is for example made of a rubbery plastic material (for example silicone); thereby, a user can push the container 2 from below, resting the mouth 4 on the flat wall 8 of the interface element 7 by exerting a slight pressure and thus quickly and simply obtain a temporary and optimal closure (i.e., without appreciable clearance and thus watertight or almost watertight) of the mouth 4. According to an alternative embodiment, the interface element 7 comprises an actual threaded plug which is configured to screw around the mouth 4 of the container 2.

The dispensing machine 1 comprises a delivery pipe 9, which ends with a delivery opening 10, and a return pipe 11 which starts with a return opening 12; both pipes 9 and 11 are arranged through the flat wall 8 of the interface element 7, i.e., the interface element 7 is crossed from side to side by the delivery pipe 9 and the return pipe 11. Thereby, when the interface element 7 is coupled to the mouth 4 of the container 2 (as illustrated in figure 2), the delivery opening 10 of the delivery pipe 9 and the return opening 12 of the return pipe 11 are both inside the container 2.

The dispensing machine 1 comprises an ionised air generator 13 which is configured to generate an ionised air flow and is arranged along the delivery pipe 9 so that in use, the ionised air flow flows along the delivery pipe 9; in other words, the delivery pipe 9 (ending with the delivery opening 10) is connected to the ionised air generator 13 to receive the ionised air flow and to emit the ionised air flow from the delivery opening 10 in use.

The ionised air generator 13 comprises (at least) a fan 14 which is arranged along the delivery pipe 9 and is configured to generate an air flow which flows through the delivery pipe 9 to exit from the delivery opening 10; in particular, the fan 14 can consist of any device capable of generating an air flow along the delivery pipe 9. Furthermore, the ionised air generator 13 comprises (at least) an ionising device 15 which is arranged along the delivery pipe 9 (preferably downstream of the fan 14) and is configured to generate cold plasma (i.e., non-thermal plasma) in the air which is conveyed along the delivery pipe 9.

The cold plasma is capable of enriching the air with molecules with greater oxidising power (O, OH, H, etc.) with respect to oxygen and can produce long-lived reactive species, for example ozone, or catalytic intermediate species, for example NO, which can further accelerate oxidation. The non-thermal plasma (also called cold plasma or non-equilibrium plasma) generated by the ionising device 15 is a type of plasma in which the electrons are not in thermodynamic equilibrium with the other species, as they are characterised by a much higher temperature with respect to the heavier species (ions and neutral species).

Preferably, a chamber 16 (generally cylindrical in shape) is arranged along the delivery pipe 9 in which the ionising device 15 is arranged. The ionising device 15 comprises a bipolar ionisation tube (or cylindrical tubular-shaped capacitor) and a generator which applies an alternating electrical voltage (for example at a frequency of 50 Hz) and of high value (for example around 3 KVolt) to the ionisation tube. The ionisation tube is structured to generate an electric field which causes a corona discharge when it is powered with a '*high voltage*' (generally between 1.5 and 4 KVolt) so as to ionise the gas molecules surrounding it.

In other words, the ionising device 15 of the ionised air generator 13 comprises the bipolar ionisation tube which is arranged inside the chamber 16 between an inlet and an outlet of the chamber 16 and is configured to produce cold plasma by generating an electric field, which causes a corona discharge, when it is powered with an alternating electric voltage, so as to ionise the gas molecules surrounding it.

The gas molecules which are located near the bipolar ionisation tube undergo an excitation phenomenon which is capable of reorganising the electronic structure of the species (atoms, molecules), producing excited species, radicals and ions. That is, the bipolar ionisation tube of the ionising device 15 ionises the gas molecules which are located near the active electrode, generating cold plasma. The bipolar ionisation tube of the ionising device 15 is in essence a generator of reactive species by NTP technology (acronym for 'Non Thermal Plasma'), and is adapted to generate a gaseous mixture with ROS reactive species (acronym for 'Reactive Oxygen Species') and RNS reactive species (acronym for 'Reactive Nitrogen Species') generally referred to as NTP gas mixture.

By way of example, the ionising device 15 could be made as described in patents US8747754B2, DE202021101578U1 and DE202021101579U1.

In the embodiment illustrated in figure 1, the dispensing machine 1 comprises an abatement device 17, which is configured to abate (neutralise, eliminate, recombine) the ozone present in the air and is arranged along the return pipe 11. According to a possible embodiment, the abatement device 17 comprises a heated chamber 18 (generally cylindrical in shape) which is arranged along the return pipe 11 and houses an electric heater 19 (for example an electric resistor) which is located between an inlet and an outlet of the chamber 18; in fact, the heat favours the reconversion of the ozone present in the air and for this purpose a catalytic body could also be provided inside the heated chamber 18.

In the embodiment illustrated in figure 1, the dispensing machine 1 comprises an absorber device 20, which is configured to absorb (capture) the ozone present in the air and is arranged along the return pipe 11 downstream of the abatement device 17; that is, the absorber device 20 absorbs the (little) residual ozone which has not been abated by the abatement device 17. According to a possible embodiment, the absorber device 20 comprises a chamber 21 (generally cylindrical in shape) which is arranged along the return pipe 11 and houses an absorber body 22 (for example having activated carbon) which is located between an inlet and an outlet of chamber 21.

According to other embodiments not illustrated, only the abatement device 17 is present along return pipe 11 (i.e., the absorber device 20 is absent) or only the absorber device 20 is present along the return pipe 11 (i.e., the abatement device 17 is absent). Alternatively, both the abatement device 17 and the absorber device 20 are absent. In other words, reduction means are present along the return pipe 11, which are configured to reduce the ozone present in the air and are provided with the abatement device 17 and/or the absorber device 20.

In the embodiment illustrated in figure 1, the delivery pipe 9 originates from a suction opening 23, which communicates with the outside and the return pipe 11 ends in an expulsion opening 24, which communicates with the outside; thereby, in use, the outside air is drawn in, is ionised, is circulated inside the container 2, is treated to abate the ozone, and is then reintroduced into the outside.

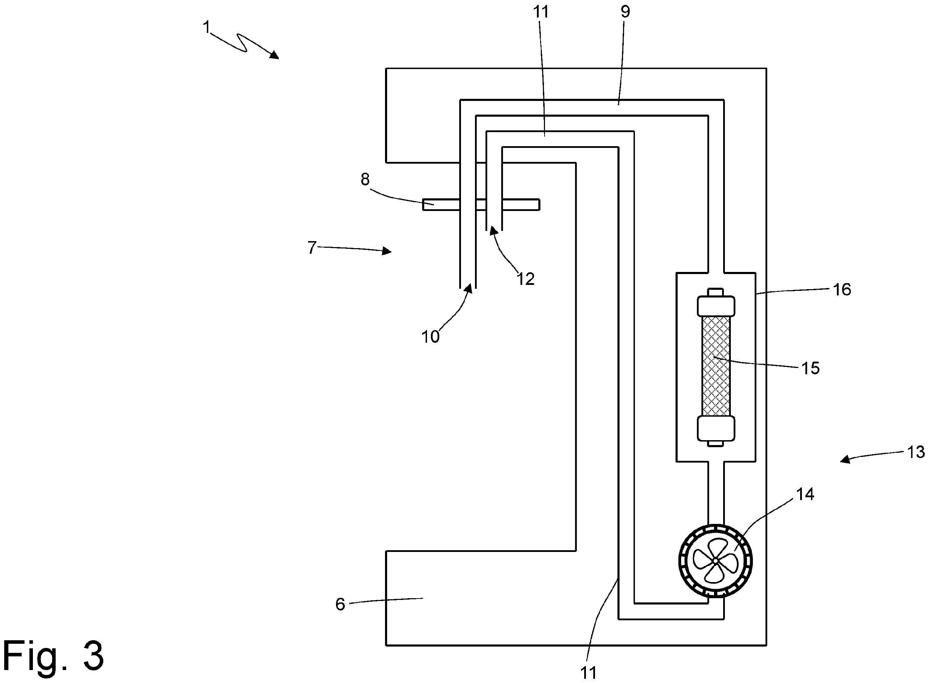

In the alternative embodiment illustrated in figure 3, an end of the delivery pipe 9 opposite the delivery opening 10 is connected to an end of the return pipe 11 opposite the return opening 12 to obtain a continuous recirculation of the same air which, in use, is ionised, is circulated inside the container 2, and is then ionised again. In this embodiment, both the abatement device 17 and the absorber device 20 are absent, as there by not introducing air into the outside, there is no need to abate the ozone.

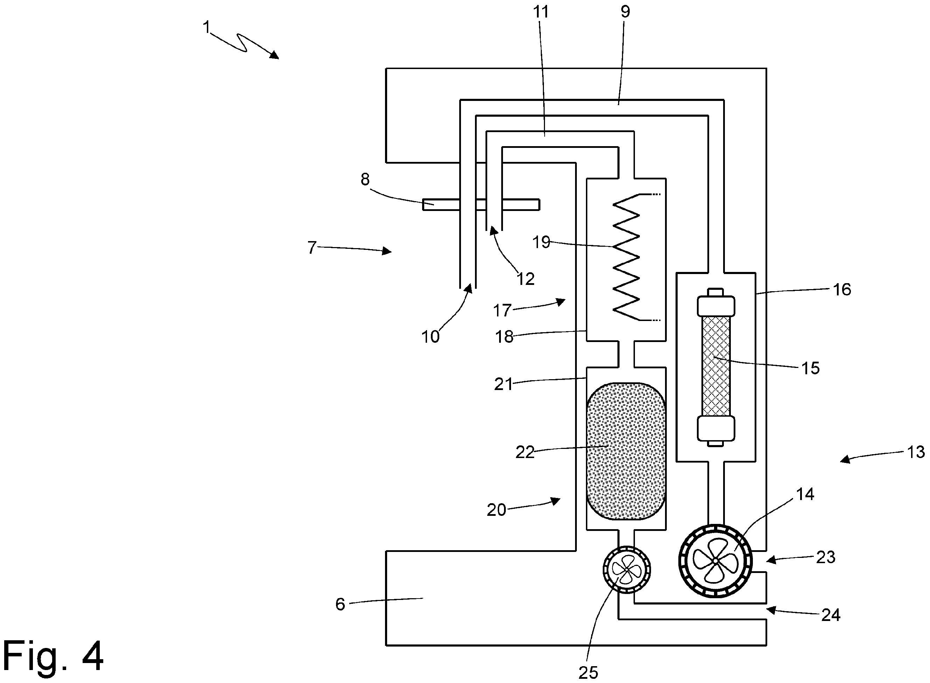

In the alternative embodiment illustrated in figure 4, the dispensing machine 1 also comprises a fan 25 which is arranged along the return pipe 11 and is configured to generate a suction through the return opening 12; thereby, the circulation of ionised air inside the container 2 is improved.

In the embodiment illustrated in figure 1, the dispensing machine 1 also comprises a pressure sensor 26 which is arranged along the delivery pipe 9 and is configured to detect the air pressure along the delivery pipe 9. A control unit 27 is configured to drive the fan 14 in feedback as a function of the reading received from the pressure sensor 26 so as to always maintain a desired pressure value in the delivery pipe 9 (and to generate an error/alarm signal if the desired pressure value cannot be maintained). The reading of the pressure sensor 26 (possibly combined with the reading of a proximity sensor arranged in the interface element 7) could also be used to determine the actual presence of a container 2 properly coupled to the interface element 7 (in the absence of a container 2 properly coupled to the interface element 7, the air pressure along the delivery pipe 9 remains relatively low even when the fan 14 is operating at full power).

Figure 2 shows the dispensing machine 1 in use with the air paths highlighted: the air from the outside is sucked in through suction opening 23 by the action of fan 14 and is then conveyed through the delivery pipe 9 to pass through the ionising device 15; the ionised air from the ionising device 15 continues along delivery pipe 9 until it exits through the delivery opening 10 and thus circulates inside the container 2. The ionised air which has circulated inside the container 2 enters (due to the overpressure generated inside the container 2 as a result of blowing ionised air through the delivery opening 10 and possibly also due to the suction action of the fan 25 if present) the return opening 12 of the return pipe 11 and then sequentially crosses the abatement device 17 and the absorber device 20 before being re-emitted into the outside through the expulsion opening 24.

According to a preferred embodiment, downstream of the interface element 7, a length of the delivery pipe 9 is different from a length of the return pipe 11 so that the delivery opening 10 is at a different height than the return pipe 11; in particular, the delivery pipe 9 can be longer than the return pipe 11 (as illustrated in the accompanying figures) or vice versa. Thereby, the ionised air emitted by the delivery opening 10 of the delivery pipe 9 tends to circulate more inside the container 2 before entering the return opening 12 of the return pipe 11.

Generally, the container 2 which is coupled to the dispensing machine 1 is empty and the function of the ionised air which is circulated inside the container 2 is to sanitise the surfaces delimiting the internal volume 3 of the container 2 to eliminate algae and microorganisms; in fact, a high concentration of oxidising species in the internal volume 3 of the container 2 which is modest produces an effective sanitising (disinfecting) effect. However, it is also possible to couple the dispensing machine 1 with a container 2 filled with a beverage (usually, but not necessarily, water) inside of which the ionised air flow emitted by the delivery opening 10 of the delivery pipe 9 is bubbled, both to sanitise (disinfect) the beverage and to enrich the beverage with active ions which, according to recent scientific publications, have a positive effect on human health.

The embodiments described herein can be combined with each other without departing from the scope of protection of the present invention.

The above described dispensing machine 3 has several advantages.

Firstly, the dispensing machine 3 described above allows the container 2 to be sanitised (disinfect) in a particularly simple, quick, effective and efficient manner.

Furthermore, the dispensing machine 3 described above is relatively simple and inexpensive to manufacture.

Finally, the operation of the dispensing machine 3 described above is completely environmentally friendly, as it does not require the use of any chemicals.

### LIST OF REFERENCE NUMBERS OF THE FIGURES

- 1: dispensing machine
- 2: container
- 3: internal volume
- 4: mouth
- 5: cap
- 6: frame
- 7: interface element
- 8: flat wall
- 9: delivery pipe
- 10: delivery opening
- 11: return pipe
- 12: return opening
- 13: ionised air generator
- 14: fan
- 15: ionising device
- 16: chamber
- 17: abatement device
- 18: chamber
- 19: electric heater
- 20: absorber device
- 21: chamber
- 22: absorbing body
- 23: suction opening
- 24: expulsion opening
- 25: fan
- 26: pressure sensor
- 27: control unit

## Claims

1. A dispensing machine (1) to dispense ionised air into a container (2) having, at the top, a mouth (4); the dispensing machine (1) comprises:
an ionised air generator (13) configured to generate an ionised air flow;
a delivery pipe (9), which ends with a delivery opening (10) and is connected to the ionised air generator (13) so as to receive the ionised air flow and emit the ionised air flow from the delivery opening (10);
a return pipe (11), which starts with a return opening (12); and
an interface element (7), which is configured to be coupled to the mouth (4) of the container (2) and is crossed by the delivery pipe (9) and by the return pipe (11) so that, when the interface element (7) is coupled to the mouth (4) of the container (2), the delivery opening (10) of the delivery pipe (9) and the return opening (12) of the return pipe (11) are both inside the container (2).

2. The dispensing machine (1) according to claim 1, wherein the ionised air generator (13) comprises a first fan (14), which is arranged along the delivery pipe (9) and is configured to generate an air flow, which flows through the delivery pipe (9) in order to flow out of the delivery opening (10).

3. The dispensing machine (1) according to claim 1 or 2 and comprising a second fan (25), which is arranged along the return pipe (11) and is configured to generate a suction through the return opening (12).

4. The dispensing machine (1) according to claim 1, 2 or 3, wherein:
the delivery pipe (9) originates from a suction opening (23), which communicates with the outside; and
the return pipe (11) ends in an expulsion opening (24), which communicates with the outside.

5. The dispensing machine (1) according to claim 1, 2 or 3, wherein an end of the delivery pipe (9) opposite the delivery opening (10) is connected to an end of the return pipe (11) opposite the return opening (12).

6. The dispensing machine (1) according to one of the claims from 1 to 5 and comprising reduction means which are configured to reduce the ozone present in the air and are arranged along the return pipe (11).

7. The dispensing machine (1) according to claim 6, wherein the reduction means are provided with an abatement device (17) which is configured to abate the ozone present in the air and is arranged along the return pipe (11).

8. The dispensing machine (1) according to claim 7, wherein the abatement device (17) comprises a heated chamber (18) provided with an electric heater (19).

9. The dispensing machine (1) according to claim 6, 7 or 8, wherein the reduction means are provided with an absorber device (20), which is configured to absorb the ozone present in the air and is arranged along the return pipe (11) .

10. The dispensing machine (1) according to one of the claims from 1 to 9, wherein the interface element (7) comprises a flat wall (8), which is elastically deformable and is crossed by the delivery pipe (9) and by the return pipe (11).

11. The dispensing machine (1) according to one of the claims from 1 to 10, wherein, downstream of the interface element (7), a length of the delivery pipe (9) is different from a length of the return pipe (11) so that the delivery opening (10) is at a different height than the return pipe (11) .

12. The dispensing machine (1) according to one of the claims from 1 to 11, wherein the ionised air generator (13) comprises:
a chamber (16);
a bipolar ionisation tube, which is arranged inside the chamber (16) between an inlet and an outlet of the chamber (16) and is configured to produce cold plasma by generating an electrical field, which causes a corona discharge, when it is powered with an electrical voltage, so as to ionise the gas molecules surrounding it; and
a generator, which applies an alternating electrical voltage to the ionisation tube.

13. A dispensing method to dispense ionised air into a container (2); the dispensing method comprises the steps of:
generating an ionised air flow by means of an ionised air generator (13);
directing the ionised air flow into a delivery pipe (9) ending with a delivery opening (10) so as to emit the ionised air flow from the delivery opening (10); and
coupling the mouth (4) of the container (2) to an interface element (7), which is crossed by the delivery pipe (9) and by a return pipe (11), which starts with a return opening (12), so that the delivery opening (10) of the delivery pipe (9) and the return opening (12) of the return pipe (11) are both inside the container (2).

14. The dispensing method according to claim 13, wherein the container (2), when it is coupled to the interface element (7), holds, on the inside, a beverage in which the ionised air flow is caused to bubble.

15. The dispensing method according to claim 13 or 14, wherein the ozone present in the air is reduced by reduction means arranged along the return pipe (11).
